# EUROPEAN PATENT SPECIFICATION

(11) **EP 3 840 820 B1**
(45) Date of publication and mention of the grant of the patent: **28.01.2026**
(21) Application number: 19772826.4
(22) Date of filing: 04.07.2019
(51) Int. Cl.: A61M 37/00

(54) **MULTIFUNCTIONAL PENCIL FOR EYEBROW STYLING**
MULTIFUNKTIONALER STIFT FÜR AUGENBRAUENSTYLING
CRAYON MULTIFONCTION POUR LA MISE EN FORME DES SOURCILS

(30) Priority: 21.08.2018 RS P20180980
(43) Date of publication of application: 30.06.2021
(73) Proprietor: Phiacademy Doo Beograd-Vozdovac, Belgrade, 11000 (RS)
(72) Inventor: BABIC, Branko, 11000 Belgrade (RS)
(74) Representative: Brkic, Zeljka
(86) International application number: PCT/IB2019/055712
(87) International publication number: WO 2020/039275

(56) References cited:
- CN-U- 202 078 646
- CN-U- 205 568 181
- CN-U- 207 605 229
- GB-A- 2 548 083
- TW-U- M 537 838

## Description

### Field of the invention

The subject invention relates to a multifunctional pencil for eyebrow styling. According to the International Patent Classification, the subject invention belongs to the tools for introducing a medium, in this case a dye, under the skin, which are designated with code A61 M 37/00.

### Technical problem

The technical problem solved by the subject invention consists of how to design a pencil for the treatment of eyebrow styling, which would contain two types of blades in itself: such as the U-shaped blade, used for drawing the hairs, and a blade in the shape of a needle, which is used to shade the space between the hairs, so that it constitutes a unique tool that would be easy to handle and that would shorten the time of work, at which the blades would be housed in the pencil itself, which would prevent contamination as the complete pencil would be sterile.

### Prior art

Even in the past, for better appearance, women began to pencil in their eyebrows and thus shape and modify them. This was originally done with eyebrow styling pencils and by tweezing and waxing. With the industry's development in terms of beauty products, the need for perfect eyebrows has also developed. So today, there are various methods and techniques for modifying and shaping eyebrows such as tattooing, dyeing, make-up, reshaping by hair removal, and the like. However, one technique that has taken the primacy in the filling and shaping eyebrows is - microblading.

Microblading is a technique for filling and shaping eyebrows that gives eyebrows fullness and natural form without additional styling and pencil shading. This technique is able to complement existing eyebrows, correct irregularities, or draw a completely new shape. It does not require removal of existing hair, but it follows the line of the natural arch of the eyebrows and fills-in empty space with an aim of achieving a beautiful contour and fullness of the eyebrows. To fill-in an eyebrow, a mineral pigment in liquid, creamy or gel form is used and applied to the area of the eyebrow. During the treatment, the pigment is inserted shallow under the skin by means of the blade and in this manner the eyebrows are drawn. At which two techniques are combined: microblading and shading. This does not fill-in the whole eyebrow, but rather hairs are drawn together with natural hairs with a dye that is not different from the existing color of natural hair, which gives eyebrows a natural appearance.

The difference between the existing styling methods, such as tattooing, in relation to the microblading technique, by application of the subject-matter invention, is in the direction, density, length and thickness of the drawn hair. Namely, tattooing is exclusively based on the filling of the brow area, whereby after a while, dye spills and irregularities can occur, and in some cases the pigment takes on a shade of cyan. Conversely, microblading takes particular care of the position of the very hair that are plotted and which are very thin, which provides a more natural look.

The tool most commonly used for drawing eyebrows with various permanent make-up methods is a small hand-held single-needle device. In practice, there are several enhanced versions of this device, however, the point is that it uses a needle that, once dipped into the pigment, penetrates into the dermal layer of the skin, resulting in the spread of dye also into the area that should not be dyed, which results in spillage and irregular blurred lines.

In microblading, a specially designed tool is used, i.e. a fine, special blade that is placed in the holder. The blade itself is made of many fine, tiny pins that are used to perform extremely delicate and precise cuts into the skin so that it may absorb the pigment that will ultimately give the appearance of natural eyebrow hairs. A blade such as this guarantees the highest possible precision of pigment implementation so far, and provides a gentle and precise contours without causing unintended side effects that occur in tattooing; conversely, all lines are streaked precisely and mimic the appearance of natural hairs.

Until now, people used holders to alternatively use blades or pins, depending on which technique is being used, i.e. whether they wanted to perform microblading or shading. Each technique requires a certain blade, which consumes time during the exchange and thus prolongs the duration of the treatment. The subject invention shortens the working time because the blades are situated in one package, meaning in one pencil, which accelerates operation due to the simplified transition from one blade to another.

In the specification of the Chinese utility model CN 207605229 U, which is considered to be the closest prior art, is disclosed a microblading pencil which consists of a one-piece body, on one side of which an opening is made, in which a corresponding microblading blade is inserted, while on its opposite side another opening is made, in which clamping mechanism with clamping sleeve and spring for holding the appropriate needle is inserted. One lid is placed on both sides of the body, which covers the needle, i.e. the blade. The mentioned pencil has the features of the preamble of claim 1. Although this pencil has a number of advantages, which include a high level of hygiene and easy handling with the pen, it also has a number of disadvantages, among which are the complex design of the clamping mechanism for holding the needle, which significantly increases the cost of making a pencil and increases the possibility of usage errors, as well as lack of hygienic applicator on it. Therefore, the user must additionally have a special hygienic applicator at his disposal, which slows down and makes it difficult to work with the pencil due to taking and disposing of this applicator. It also does not provide for the penetration of sterile gas to the cosmetic implement such as a pen, a brush or a microblading blade.

In the specification of the Chinese utility model CN 205568181 U is disclosed a multi-functional eyebrow pencil that includes a pen-holder for holding the appropriate pen covered with a lid. The other end of the pen-holder is equipped with the brush-holder that is in turn equipped with the brush which is also covered with another lid. A bottle containing eyebrow dye is arranged inside the cavity of the pen-holder. Although the structure of the mentioned multi-functional eyebrow pencil is simple and convenient for use, nevertheless it is not suitable for microblading. It also does not provide for the penetration of sterile gas to the cosmetic implement such as a pen, a brush or a microblading blade. It is also lacking a hygienic applicator thereon. Therefore, the user must additionally have a special hygienic applicator at his/her disposal, which slows down and makes it difficult to work with the pencil due to taking and disposing of this applicator.

### Summary of the Invention

The above-defined technical problem is solved by a a multifunctional pencil for eyebrow styling, as defined by independent claim 1 and subsequent dependent claims 2 - 6, that carries a microblading blade, a shadow blade (i.e.shading needle), and a hygienic applicator. The multifunctional pencil for eyebrow styling comprises a cylindrical lid and a middle part. The middle part has one opening on its front end and one opening on its rear end. The cylindrical lid is placed on the front end of the middle part and a microblading blade is placed in the opening in the front end of the middle part. Furthermore, the pencil further comprises a rear part, which has a shading needle, which is located on the conical protrusion on the front end of the rear part and intended for inserting into the opening in the rear end of the middle part when the rear part of the pencil is placed on the middle part thereof. The rear part further has a rectangular hygienic applicator located on its rear end. The lid has an opening in its central part for the penetration of sterile gas to the blade.

The lid is cylindrical in shape, made of transparent plastic so that the operator would have a visual overview of the blade itself. On the front of the lid there is a round cone opening for penetration of the sterilized gas to the blade, while the rear part of the lid is round-shaped with a ring around the entire inner circumference, the function of which is to position the lid for the middle part of the pencil.

The middle part of the pencil is barrel-shaped with two cylindrical surfaces that give symmetry to the piece and serve as an aesthetic and functional connection od this part of the pencil with the rear part of the pencil. Around its entire surface, there are ball-shaped indents for easier operation of the tool itself, which gives the operator greater freedom of operation, as well as more precise work and safer hold. The middle part of the pencil has two openings, from the front and from the back. The front opening of the middle part is used for setting the microblading blades, while the back opening serves for the positioning and binding of the rear part of the pencil, i.e. insertion of the needle. The front opening of the middle part may differ in the depth of the opening, depending on which blade is to be used, the golden blade or the black blade.

The rear part of the pencil is of a more complex shape, which is a variable cylindrical shape on the front end thereof that binds to the middle part, while the rear end thereof is of the rectangular shape on which there is a hygienic applicator in the form of three ribs along the longitudinal axis of the pencil with a slight curvature in direction opposite to the longitudinal axis of the pencil, which covers about one-eighth of the pencil. On the front end of the rear part of the pencil there is a conical protrusion with a cut flat part for positioning of the middle part of the pencil, on which the needle is located.

The blade used in the subject invention, which is located on the front end of the middle part of the pencil, is U-shaped and contains 18 tiny needles, the so-called pins. They serve to draw hairs in eyebrows and come in the form of a black or golden blade. Both blades can create hairs that look quite natural, while the main difference is that in the golden blade the pins are extremely sharp, making it easier to penetrate into the skin, even if the skin is thick and problematic; also, it produces extremely thin hairs. Its thinner pins allow for greater flexibility, and a greater difference between the hairs facilitates quicker filling-in with pigment.

The needle used in the subject invention, which is located on the front end of the rear part of the pencil, consists of three fine pins that are arranged in a circular manner. This needle is used to shade the space between the hairs and is ideal for shading small surfaces. The very material from which the pencil is made is of high quality. At fabrication of the pencil, a special mixture (Addiflex^{®}) with polyphenols is used, which is susceptible to degradation without harmful effects on the environment. When released into the environment, this material degrades spontaneously and assimilates into it without any negative environmental effects. The material itself has been tested and is suitable for use in the fabrication of a pencil is meant to be used in direct contact with human skin.

The design of the pencil is distinguished, in comparison with the existing universal holders, among other things in that that all blades, for microblading and for shading, are situated in one package and in that preliminary sterilization of the universal holder is not necessary, as is neither the opening of additional blades. In this way, operation is accelerated due to the simplicity of switching between one blade and another.

### Brief description the Drawings

An embodiment of the pencil according to the invention is described in detail below and is shown in the enclosed figures. They show:
Figure 1a shows a horizontal projection of a pencil with a lid,
Figure 1b shows a side view of the pencil with the lid,
Figure 2 shows a perspective view of the disassembled pencil,
Figure 3a shows a longitudinal section of the lid,
Figure 3b shows the rear view of the lid,

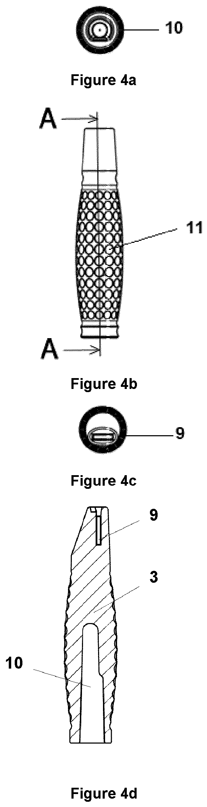

Figure 4a shows the rear view of the middle part of the pencil,
Figure 4b shows a horizontal projection of the middle part of the pencil,
Figure 4c shows the front view of the middle part of the pencil,
Figure 4d shows a longitudinal section in a vertical plane perpendicular to the longitudinal axis of the middle part with openings for a blade and a needle,
Figure 5a shows a perspective view of the rear part of the pencil,
Figure 5b shows a longitudinal section of the rear part of the pencil,
Figure 5c shows a horizontal projection of a hygienic applicator,
Figure 5d shows a perspective view of the hygienic applicator,
Figure 6a shows a horizontal projection of the blade,
Figure 6b shows the side view of the blade,
Figure 6c shows a horizontal projection of the needle, and
Figure 6d shows a perspective view of the needle.

### Detailed description of the Invention

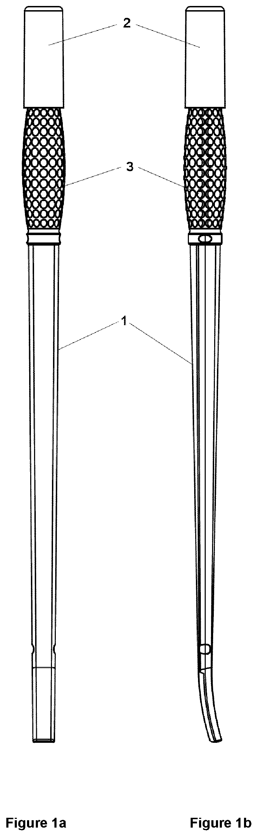

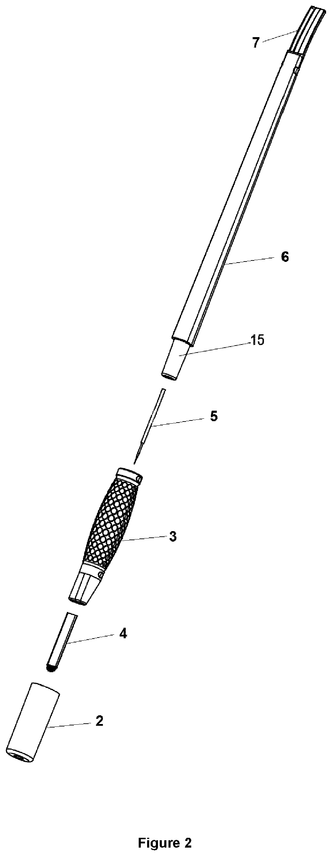

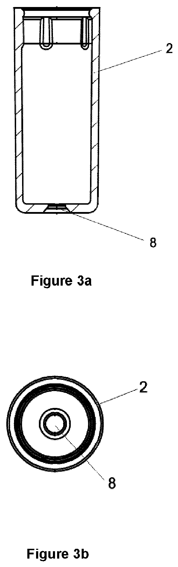

As can be seen from the Figures, and in particular from Figs. 1a, 1b and 2, the multifunctional pencil (1) for eyebrow styling comprises a cylindrical lid (2), which is placed on the front end of the middle part (3) of the pencil (1), which has a microblading blade (4) on its front end and on the rear end thereof there is an opening (10) for inserting a shading needle (5) located on the conical protrusion (15) on the front end of the rear part (6), which on its rear end has a rectangular hygienic applicator (7) which has three ribs. Referring to Figures 3a and 3b, viewed from above, the lid (2) itself, which is circular in shape, in its central part has a round opening (8) for penetration of sterile gas to the blade (4), wherein the lid (2) is connected to the front end (9) of the middle part (3) by means of a ring, which is located along the entire inner circumference of the lid (2).

As can be seen from the Figures, the middle part (3) of the pencil (1) is of a barrel-shape with two cylindrical surfaces that give symmetry to the middle part (3). Throughout its entire surface, the middle part (3) has ball-shaped indents (11) for easier grip of pencil (1) during use. The cylindrical front end of the middle part (3) of the pencil is tapered so that the tip of the front end narrows by one-half of the width of the middle part (3) itself, and passes into an elliptical shape. The opening (9) on the front end of the middle part (3), when viewed from above, is elliptical in shape and is used to mount the microblading blades (4), at which the aperture itself for the positioning of the blade is of a rectangular shape. Whereas the opening (10) at the rear end of the middle part (3), when viewed from above, is circular in shape, with one part of the circle is cut flat. It serves to position the needle (5) and for connection of the rear part (6). When looking at the cross- section of the middle part (3), the depth of the rear end with the flattened side on one part occupies one-half of the height of the middle part (3), while the depth of the front end corresponds to the length of the blade (4), which can be either 15 mm or 20 mm, depending on which blade is used.

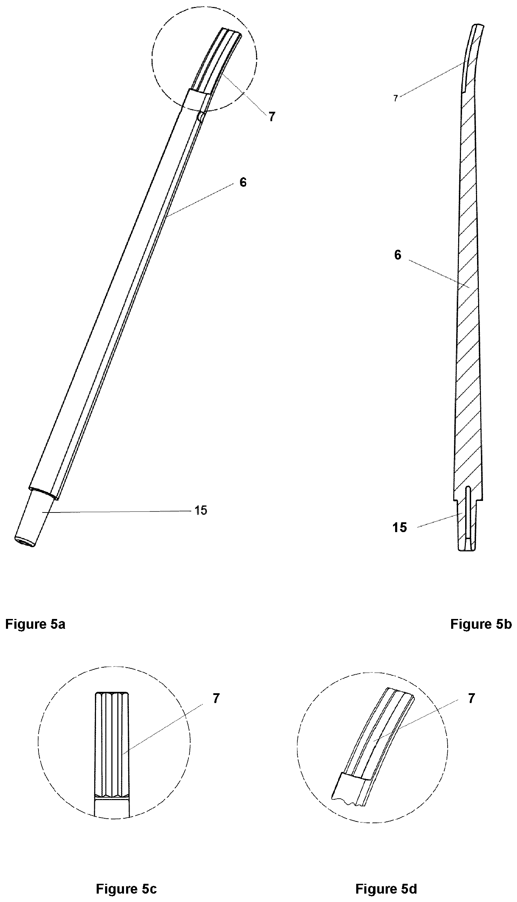

As can be seen from Figures 5a-5d, the front end of the rear part (6) of the pencil (1), behind the conical protrusion (15) where the needle (5) is located, is cylindrical in shape, wherein the needle (5) is located and connects it to the middle part (3) of the pencil (1), while on the rear end it narrows and turns into a rectangular shape with a slight curvature in direction opposite to the longitudinal axis of the pencil (1). At the rear end of the rear part (6) of the pencil (1), there is a hygienic applicator (7), which is also rectangular in shape and on which there are three ribs along the longitudinal axis of the pencil (1). The conical protrusion (15) of the rear part (6) of the pencil, which has the opening in which the needle (5) is inserted, is of a smaller diameter than the diameter of the opening (10) in the rear end of the middle part (3), so that it can bond, i.e. fit into the middle part (3) and thus connect the middle part (3) of the pencil (1) with the rear part (6) of the pencil (1).

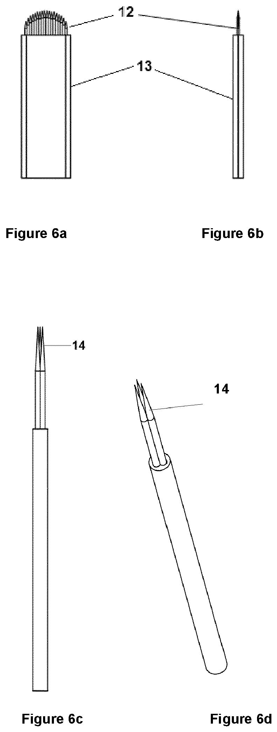

The appearance of the pin (4) which is used for microblading, as can be seen from Figs. 6a and 6b, is such that it consists of tiny pins (12), which are of different lengths and which are arranged one next to the other, creating a U-shape. They are made of high quality stainless steel, and the pin holder (13) is made of plastic. The needle (5) that is used for shading, according to Fig. 6b, consists of three needles (14) that are arranged circularly, which in this way form a thicker needle. The material from which the needles are made is also of high quality stainless steel.

## Claims

1. A multifunctional pencil for eyebrow styling comprising a cylindrical lid (2) and a middle part (3), which has one opening in its front end and one opening in its rear end, wherein the cylindrical lid (2) is placed on the front end of the middle part (3) and a microblading blade (4) is placed in the opening in the front end of the middle part (3), **characterized in that** the pencil (1) further comprises a rear part (6), which has a shading needle (5), which is located on the conical protrusion (15) on the front end of the rear part (6) and which is intended for inserting into the opening in the rear end of the middle part (3) when the rear part (6) of the pencil (1) is placed on the middle part (3) thereof, and wherein the rear part (6) further has a rectangular hygienic applicator (7) located on its rear end, wherein the lid (2) has an opening (8) in its central part for the penetration of sterile gas to the blade (4).

2. The multifunctional pencil for eyebrow styling according to claim 1, **characterized in that** the middle part (3) of the pencil (1), which is barrel-shaped, has ball-shaped indents on its surface.

3. The multifunctional pencil for eyebrow styling according to claim 1 or 2, **characterized in that** the front end of the middle part (3), on which the blade (4) is placed, is elliptical in shape.

4. The multifunctional pencil for eyebrow styling according to claim 1 or 2, **characterized in that** the rear end of the middle part (3), into which the needle (5) is inserted when the rear part (6) of the pencil (1) is placed on the middle part (3) of the pencil (1), is circular in shape.

5. The multifunctional pencil for eyebrow styling according to claim 1, **characterized in that** the rear part (6) is cylindrical in shape on the front end, behind the conical protrusion (15), while it tapers on the rear end and turns into a rectangular shape with a slight curvature in the direction opposite to the longitudinal axis of the pencil (1).

6. The multifunctional pencil for eyebrow styling according to claim 1, **characterized in that** the hygienic applicator (7) is of a rectangular shape with three ribs along the longitudinal axis of the pencil (1).

## Patentansprüche

1. Multifunktionaler Augenbrauenstift, bestehend aus einem zylindrischen Kappe (2) und einem Mittelteil (3) mit je einer Öffnung an seinem vorderen und hinteren Ende, wobei der zylindrische Kappe (2) auf dem vorderen Ende des Mittelteils (3) aufgesetzt ist und eine Mikroblading-Klinge (4) in die Öffnung am vorderen Ende des Mittelteils (3) eingesetzt wird, **dadurch gekennzeichnet, dass** der Stift (1) umfasst ferner ein Teil (6) mit einer Schattierungsnadel (5) an der konischen Kegel (15) am vorderen Ende des hinteren Teils (6) befindet und dazu bestimmt ist, in die Öffnung am hinteren Ende des Mittelteils (3) eingeführt zu werden, wenn der hintere Teil (6) des Stifts (1) auf dessen Mittelteil (3) gesetzt wird, wobei der hintere Teil (6) ferner einen rechteckigen Hygieneapplikator (7) an seinem hinteren Ende aufweist, wobei der Kappe (2) in seinem Mittelteil eine Kegels (8) zum Eindringen von sterilem Gas zur Klinge (4) aufweist.

2. Multifunktionaler Augenbrauenstift nach Anspruch 1, **dadurch gekennzeichnet, dass** der zylinderförmige Mittelteil (3) des Stifts (1) kugelförmige Vertiefungen auf seiner Oberfläche aufweist.

3. Multifunktionaler Augenbrauenstift nach Anspruch 1 oder 2, **dadurch gekennzeichnet, dass** das vordere Ende des Mittelteils (3), auf dem die Klinge (4) angebracht ist, elliptisch geformt ist.

4. Multifunktionaler Augenbrauenstift nach Anspruch 1 oder 2, **dadurch gekennzeichnet, dass** das hintere Ende des Mittelteils (3), in das die Nadel (5) eingeführt wird, wenn der hintere Teil (6) des Stifts (1) auf den Mittelteil (3) des Stifts (1) aufgesetzt wird, kreisförmig ist.

5. Multifunktionaler Augenbrauenstift nach Anspruch 1, **dadurch gekennzeichnet, dass** der hintere Teil (6) an seinem vorderen Ende hinter dem konischen Kegel (15) zylindrisch geformt ist, sich aber zum hinteren Ende hin verjüngt und in eine rechteckige Form mit einer leichten Krümmung in der der Längsachse des Stifts (1) entgegengesetzten Richtung übergeht.

6. Multifunktionaler Augenbrauenstift nach Anspruch 1, **dadurch gekennzeichnet, dass** der rechteckige Hygieneapplikator (7) drei Rippen aufweist, die parallel zur Längsachse des Stifts (1) verlaufen.

## Revendications

1. Le crayon à sourcils multifonctionnel pour le stylisme des sourcils comprenant un capuchon cylindrique (2) et une partie centrale (3), dotée d'une ouverture à son extrémité avant et d'une autre à son extrémité arrière. Le capuchon cylindrique (2) est placé sur l'extrémité avant de la partie centrale (3), et une lame de microblading (4) est placée dans l'ouverture située à l'extrémité avant de la partie centrale (3), **se caractérise par le fait que** le crayon (1) comprend en outre une partie arrière (6), qui comporte une aiguille à ombrer (5), située sur la protubérance conique (15) à l'extrémité avant de la partie arrière (6) et destinée à être insérée dans l'ouverture située à l'extrémité arrière de la partie centrale (3) lorsque la partie arrière (6) du crayon (1) est placée sur la partie centrale (3), et dans laquelle la partie arrière (6) comporte en outre un applicateur hygiénique rectangulaire (7) situé à son extrémité arrière, le couvercle (2) comportant une ouverture (8) dans sa partie centrale pour la pénétration de gaz stérile jusqu'à la lame (4).

2. Le crayon à sourcils multifonctionnel, conformément à l'exigence 1, **se caractérise par le fait que** la partie centrale (3) du crayon (1), de forme cylindrique, présente des encoches sphériques sur sa surface.

3. Le crayon à sourcils multifonctionnel, conformément à l'exigence 1 ou 2, **se caractérise par le fait que** la partie centrale (3), sur laquelle est placée la lame (4), est de forme elliptique.

4. Le crayon à sourcils multifonctionnel, conformément à l'exigence 1 ou 2, **se caractérise par le fait que** l'avant de la partie centrale (3), dans laquelle l'aiguille (5) est insérée lorsque la partie arrière (6) du crayon (1) est placée sur la partie centrale (3) du crayon (1), est de forme circulaire.

5. Le crayon à sourcils multifonctionnel, conformément à l'exigence 1, **se caractérise par le fait que** la partie arrière (6) est de forme cylindrique à l'extrémité avant, derrière la protubérance conique (15), tandis qu'elle s'effile à l'extrémité arrière et se transforme en une forme rectangulaire avec une légère courbure dans la direction opposée à l'axe longitudinal du crayon (1).

6. Le crayon à sourcils multifonctionnel, conformément à l'exigence 1, **se caractérise par le fait que** l'applicateur hygiénique rectangulaire (7) possède trois nervures parallèles à l'axe longitudinal du crayon (1).
